# FASCICULE DE BREVET EUROPEEN

(11) **EP 0 975 315 B1**
(45) Date de publication et mention de la délivrance du brevet: **14.08.2002**
(21) Numéro de dépôt: 98963624.6
(22) Date de dépôt: 23.12.1998
(51) Int. Cl.: A61K 7/06, A61K 7/48

(54) **COMPOSITION COSMETIQUE A BASE DE POLYURETHANNES ASSOCIATIFS ET DE SILICONES QUATERNAIRES**
KOSMETISCHE ZUBEREITUNG AUF DER BASIS VON ASSOZIATIVEN POLYURETHANEN UND QUARTäREN SILICONEN
COSMETIC COMPOSITION BASED ON ASSOCIATIVE POLYURETHANES AND QUATERNARY SILICONES

(30) Priorité: 13.02.1998 FR 9801776
(43) Date de publication de la demande: 02.02.2000
(73) Titulaire: L'OREAL, 75008 Paris (FR)
(72) Inventeur: DUPUIS, Christine, F-75018 Paris (FR)
(74) Mandataire: Miszputen, Laurent
(86) Numéro de dépôt international: FR9802865
(87) Numéro de publication internationale: WO9940892

(56) Documents cités:
- EP-A- 0 282 720
- EP-A- 0 745 373
- DE-C- 3 719 086
- FR-A- 2 738 835
- FR-A- 2 758 262
- US-A- 5 294 692
- MA Z ET AL: "PHASE BEHAVIORS AND FILM PROPERTIES OF DISPERSIONS AND COATINGS CONTAINING ASSOCIATIVE AND CONVENTIONAL THICKENERS" JOURNAL OF APPLIED POLYMER SCIENCE, vol. 49, no. 9, 5 septembre 1993, pages 1509-1527, XP000461142

## Description

La présente invention concerne des compositions cosmétiques contenant un nouveau système épaississant à base de polyuréthannes associatifs, ainsi que leur utilisation notamment en tant que gels coiffants ou gels de soin capillaire non rincés.

L'épaississement et/ou la gélification de milieux aqueux par des polymères est depuis longtemps un important de sujet de recherche cosmétique. L'obtention d'un effet d'épaississement intéressant par un polymère hydrosoluble suppose généralement une masse molaire élevée et un volume hydrodynamique important. La gélification d'un milieu aqueux est alors considérée comme le résultat d'un réseau polymère tridimensionnel obtenu par réticulation de polymères linéaires ou par copolymérisation de monomères bifonctionnels et polyfonctionnels. L'utilisation de tels polymères de masse molaire très élevée pose cependant un certain nombre de problèmes tels que la texture peu agréable et la difficulté d'étalement des gels obtenus.

Une approche intéressante a consisté à utiliser comme épaississants, des polymères capables de s'associer réversiblement entre eux ou avec d'autres molécules ou particules. Cette association physique donne lieu à des systèmes macromoléculaires thixotropes ou rhéofluidifiables, c'est-à-dire des systèmes dont la viscosité dépend des forces de cisaillement auxquelles ils sont soumis.

De tels polymères capables de s'associer réversiblement entre eux ou avec d'autres molécules sont appelés "polymères associatifs". Les forces d'interactions en jeu peuvent être de nature très différente par exemple de nature électrostatique, de type liaisons hydrogène ou des interactions hydrophobes.

Un cas particulier de polymères associatifs sont des polymères amphiphiles, c'est-à-dire des polymères comportant une ou plusieurs parties hydrophiles qui les rendent solubles dans l'eau et une ou plusieurs zones hydrophobes par lesquelles les polymères interagissent et se rassemblent entre eux ou avec d'autres molécules.

Le document US-A-5 294 692 décrit de tels polymères associatifs à base d'uréthannes et leur propriété d'agir comme agent épaississant.

Il est connu de préparer des compositions capillaires sous forme de gel utilisant, comme système épaississant, de tels polymères amphiphiles associatifs, en conjonction avec des agents tensioactifs. On pense que les propriétés rhéologiques intéressantes des gels ainsi obtenus résultent de la formation de micelles mixtes formées par les agents tensioactifs et par les parties hydrophobes des polymères amphiphiles, ces micelles constituant une multitude de points de réticulation physique.

Cependant, ces compositions à base de polymères associatifs et d'agents tensioactifs n'ont pas toujours les propriétés cosmétiques souhaitées. Ainsi, la présence d'agents tensioactifs, même en faibles quantités, peut modifier de façon indésirable les propriétés cosmétiques desdites compositions, telles que les propriétés d'application ou de toucher après séchage. Par ailleurs, notamment dans le domaine des gels de coiffage ou de soin non rincés, il est important de pouvoir répartir uniformément le produit sur l'ensemble de la chevelure de manière à éviter les surcharges et les défauts cosmétiques qui en résultent.

La demande de brevet français n°2 738 835 porte sur des compositions cosmétiques aqueuses épaissies au moyen d'au moins un polymère amphiphile associé à au moins une protéine à groupement hydrophobe.

On a découvert à présent qu'il était possible d'obtenir un bon effet épaississant, voire gélifiant, et des propriétés cosmétiques intéressantes en associant des polyuréthannes amphiphiles associatifs à des silicones à groupements ammonium quaternaire.

Le gel obtenu par cette association a une texture très fondante et est agréable à appliquer. Le toucher final sur cheveux séchés est plus agréable et moins chargé. Le gel a par ailleurs un excellent pouvoir coitfant.

Un objet de la présente invention est donc une composition cosmétique comprenant au moins un polyuréthanne non ionique associatif en combinaison avec au moins une silicone à groupements ammonium quaternaire.

Un autre objet de la présente invention est l'utilisation de la combinaison d'au moins un polyuréthanne non ionique associatif et d'au moins une silicone à groupements ammonium quaternaire en tant que système épaississant, notamment pour la fabrication de gels de coiffage et de soin capillaire non rincés.

Un troisième objet de l'invention est un procédé de traitement cosmétique des cheveux par les compositions cosmétiques comprenant au moins un polyuréthanne associatif non ionique et au moins une silicone à groupements ammonium quaternaire.

D'autres objets apparaîtront à la lecture de la description et des exemples qui suivront.

Les compositions cosmétiques conformes à l'invention sont essentiellement caractérisées par le fait qu'elles contiennent, dans un milieu cosmétiquement acceptable,
(A) au moins un polyuréthanne associatif non ionique amphiphile et
(B) au moins une silicone à groupements ammonium quaternaire.

Les polyuréthannes associatifs non ioniques utilisés dans la présente invention correspondent de préférence à la formule générale 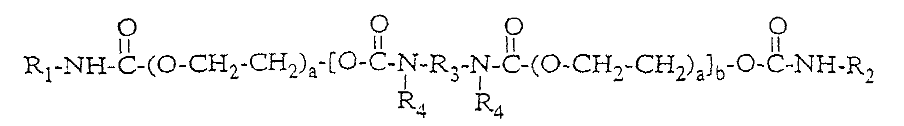 dans laquelle
au moins un des radicaux R₁ et R₂ représente un groupe alkyle supérieur en C₈₋₁₈ et l'autre, le cas échéant, un groupe alkyle inférieur en C_{1- 6}, de préférence un groupe alkyle inférieur en C₁₋₄,
R₃ représente un radical hydrocarboné en C₄₋₃₆, de préférence en C₆₋₁₀,
R₄ représente un atome d'hydrogène ou un radical alkyle en C₁₋₆, de préférence un atome d'hydrogène,
a varie indépendamment de 90 à 600, et
b vaut de 1 à 4.

On entend par groupe alkyle inférieur en C₁₋₆, selon l'invention, un groupe alkyle à chaîne linéaire ou ramifiée comportant de 1 à 6 atomes de carbone, tel que les radicaux méthyle, éthyle, *n*-propyle, *n*-butyle,
*n*-pentyle et *n*-hexyle ainsi que les isomères ramifiés correspondants.

Conformément à l'invention, les groupes alkyle supérieurs en
C₈₋₁₈ désignent des groupes alkyle à chaîne linéaire ou ramifiée comportant de 8 à 18 atomes de carbone, tels que les radicaux octyle, nonyle, décyle, undécyle, dodécyle, tridécyle, tétradécyle, pentadécyle, hexadécyle, heptadécyle et octadécyle.

Dans un mode de réalisation préféré, un seul des radicaux R₁ et R₂ représente une chaîne alkyle supérieure en C₈₋₁₈ et l'autre représente un groupe alkyle inférieur en C₁₋₆.

On utilise en particulier un polyuréthanne associatif dans lequel un des radicaux en a-W représente un groupe octadécyle et l'autre un groupe méthyle.

Les polyuréthannes associatifs utilisés dans les compositions de la présente invention sont utilisés sous forme de solution ou suspension aqueuse contenant éventuellement une certaine quantité d'amidon soluble. Cet amidon peut être n'importe quel amidon extrait de sources naturelles, tel que l'amidon de blé, de maïs, de riz, de pomme de terre etc., et qui a été modifié par voie chimique, enzymatique ou microbiologique de manière à être soluble dans l'eau.

Un polymère préféré est commercialisé par la Société Rohm & Haas sous la dénomination ACRYSOL 46. Il s'agit d'un polyuréthanne obtenu par condensation d'hexaméthylènediisocyanate et de polyéthylèneglycol, et portant à ses extrémités respectivement en moyenne un résidu méthyle et un résidu octadécyle. Ce polymère se présente sous forme d'une solution aqueuse à 15 % en poids de matière active polyuréthanne contenant, en plus, de 3 - 5 % d'une matrice d'amidon modifié par voie enzymatique.

Conformément à l'invention, on entend par "silicone à groupements ammonium quaternaire" toute silicone comportant un ou plusieurs groupements ammonium quaternaire. Ces groupements ammonium quaternaire peuvent être liés en position a-W ou sous forme de groupements latéraux. Ils peuvent être liées directement au squelette polysiloxane ou peuvent être portés par des chaînes hydrocarbonées, en particulier des chaînes polyoxyalkylénées.

Selon l'invention, on entend par silicone, en conformité avec l'acceptation générale, tout polymère ayant une structure basée sur l'alternance d'atomes de silicium et d'oxygène, reliés entre eux par des liaisons dites liaisons siloxane (-Si-O-Si-), et caractérisée en outre par l'existence de liaisons silicium-carbone. Ces silicones, ou polysiloxanes, sont généralement obtenues par polycondensation de silanes convenablement fonctionnalisés. Les radicaux hydrocarbonés les plus courants portés par les atomes de silicium sont les radicaux alkyle inférieurs, en particulier méthyle, les radicaux fluoroalkyles, les radicaux aryle et en particulier phényle.

Les silicones à groupements ammonium quaternaire de la présente invention sont par exemple choisies parmi les composés correspondants aux formules générales suivantes : 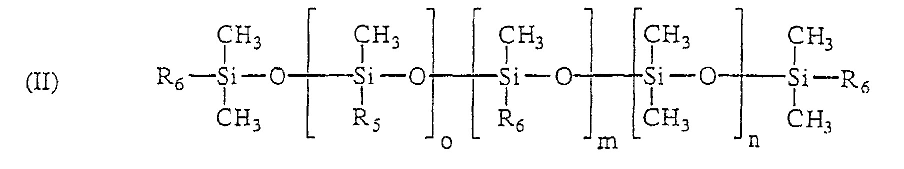 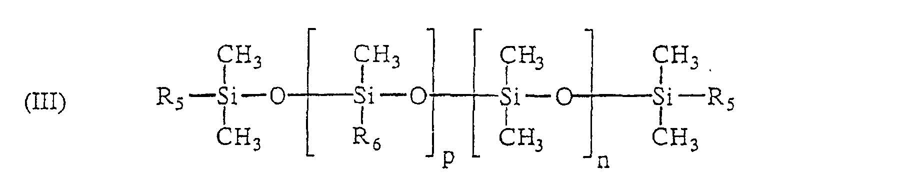 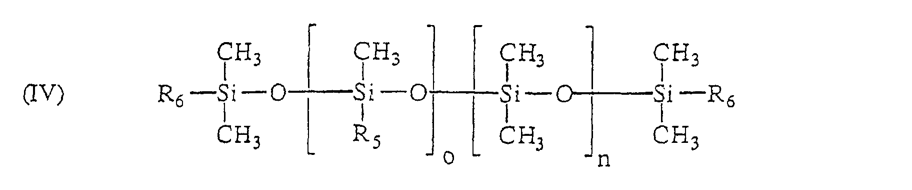 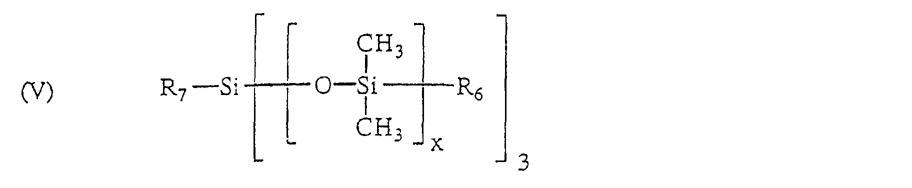 formules dans lesquelles
- les radicaux R₅, identiques ou différents, représentent des radicaux alkyle en C₁₋₃₀, linéaires ou ramifiés, ou phényle;
- les radicaux R₆, identiques ou différents, représentent un groupe -CₑH₂ₑ-O-(C₂H₄O)_{c}-(C₃H₆O)_{d}-R₈ ou -CₑH₂ₑ-O-(C₄H₈O)_{c}-R₈,
- les radicaux R₇, identiques ou différents, désignent un radical alkyle en C₁₋₁₂, linéaire ou ramifié, et de préférence le radical méthyle;
- les radicaux R₈, identiques ou différents, représentent un groupe 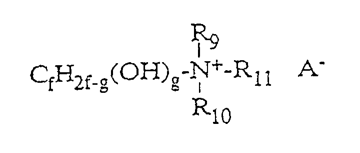
- les radicaux R₁₁ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZᵢCOR₁₂;
- R₉, R₁₀ et R₁₂, identiques ou différents, représentent des radicaux alkyle en C₁₋₂₂ ou alcényle en C₂₋₂₂, linéaires ou ramifiés, portant éventuellement un ou plusieurs groupements OH, ou R₁₀ peut former avec une partie de R₁₁ un cycle imidazoline:
   c varie de 0 à 50,
   d varie de 0 à 50,
   e et f varient indépendamment de 0 à 4,
   m varie de 0 à 20,
   n varie de 0 à 500,
   o varie de 0 à 20,
   p varie de 1 à 50,
   x varie de 1 à 100,
   g varie de 0 à 2,
   h varie de 1 à 4
   Z représente un atome d'oxygène ou d'azote,
   i vaut 0 ou 1, et

A⁻ représente un ion halogénure ou un anion organique de type carboxylate, phosphate, alcanesulfate ou sulfate.

De telles silicones sont commercialisées par exemple par la société GOLDSCHMIDT sous les dénominations ABIL QUAT 3272, ABIL B 9905, ABIL QUAT 3474 et ABIL K 3270, par la société LIPO FRANCE sous les dénominations SILQUAT Q-100, BIOSIL BASICS CETYLSIL, SILQUAT Q-200 WS, SILQUAT AX, SILQUAT AC, SILQUAT AD et SILQUAT AM tous fabriqués par la société SILTECH, par la société OSI sous la dénomination MAGNASOFT EXHAUST et SILSOFT C-880 et par la société UCIB sous les dénominations PECOSIL 14-PQ et PECOSIL 36-PQ (fabricant PHOENIX CHEMICAL).

Ces silicones sont notamment décrites dans les brevets EP 530 974, DE 3 719 086, DE 3 705 121, EP 617 607 et EP 714 654.

Les silicones à groupements ammonium quaternaire utilisées conformément à l'invention peuvent se présenter sous forme de solutions aqueuses ou éventuellement sous forme de dispersions ou d'émulsions dans l'eau.

Les silicones à groupements ammonium quaternaire et les poly-uréthannes associatifs sont utilisés en des quantités suffisantes pour obtenir un épaississement ou une gélification satisfaisants du milieu aqueux.

On utilise notamment une quantité de polyuréthannes associatifs comprise entre 0,1 et 10 % en poids et, de préférence, entre 0,5 et 5 % en poids en termes de matière active rapportée au poids total de la composition.

Dans les compositions de la présente invention, la ou les silicones à groupements ammonium quaternaire sont présentes à raison de 0,01 à
10 % en poids, de préférence à raison de 0,1 à 5 % en poids de matière active par rapport au poids total de la composition.

On utilise de préférence les silicones en une quantité telle que le rapport pondéral silicone/polyuréthanne amphiphile soit compris entre 0,1 et 10.

Le milieu cosmétiquement acceptable est constitué de préférence d'eau et peut contenir en outre des solvants cosmétiquement acceptables, par exemple des monoalcools inférieurs tels que l'éthanol ou l'isopropanol, des glycols tels que le diéthylèneglycol, des éthers de glycols tels que les alkyl-éthers d'éthylèneglycol ou de diéthylèneglycol, ou encore des esters d'acides gras, tous ces solvants étant utilisés seuls ou sous forme de mélange.

Les gels coiffants ou de soins peuvent contenir en outre un ou plusieurs additifs utilisés habituellement dans de telles compositions capillaires. On peut citer à titre d'exemple les parfums, colorants, conservateurs, filtres solaires, vitamines, agents régulateurs de pH etc. Il est bien entendu que le choix de ces composés doit tenir compte d'éventuelles interactions avec le système épaississant. L'homme du métier veillera à ce que l'adjonction de ces additifs n'aura pas d'influence défavorable sur les propriétés avantageuses des compositions obtenues grâce à la présente invention.

Un procédé de traitement cosmétique des cheveux préféré, selon l'invention, consiste à appliquer et à répartir de façon homogène les compositions définies ci-dessus sur les cheveux et à sécher les cheveux ainsi traités sans les rincer.

Il est bien entendu que la description qui précède n'a été donnée qu'à titre purement illustratif et non limitatif et que des variantes ou des modifications peuvent y être apportées dans le cadre de la présente invention.

Les exemples suivants sont destinés à illustrer l'invention sans pour autant présenter un caractère limitatif.

### Exemple 1

On prépare les compositions aqueuses suivantes comprenant 2 % en poids de matière active de polyuréthanne associatif et 1 % de matière active de silicones à groupements ammonium quaternaire ou de silicones cationiques.

| polyuréthanne associatif | silicone | nature de la silicone | consistance de la composition obtenue |
|---|---|---|---|
| Acrysol 46 | Abil Quat 3272 | quaternisée | gel |
| Acrysol 46 | Silquat Q-100 | quaternisée | gel |
| Acrysol 46 | Biosil Basic Cetylsil | quaternisée | gel |
| Acrysol 46 | SLM 23056/1 | non quaternisée | solution fluide |
| Acrysol 46 | Finish CT 110E | non quaternisée | solution fluide |
| Acrysol 46 | aucune | - | solution fluide |

Les silicones utilisées sont les suivantes :
- ABIL QUAT 3272 est un polydiméthylsiloxane à groupements acétate d'ammonium quaternaire à chaîne grasse de coco en a et w (solution à 50 % dans du propylèneglycol) commercialisé par la société GOLDSCHMIDT;
- SILQUAT Q-100 est un polydiméthylsiloxane à groupements chlorure de propyloxy-hydroxy-2-propyltriméthylammonium (solution alcoolique à 70 %) commercialisé par la société LIPO FRANCE;
- BIOSIL BASICS CETYLSIL est un polydiméthylsiloxane oxyéthyléné à groupements ester phtalique/chlorure de cétyltriéthylammonium (solution aqueuse à 40 %) commercialisé par la société SILTECH;
- FINISH CT110 E est un polydiméthyl/méthylcyclohexylaminopropylsiloxane (en émulsion non ionique) commercialisé par la société WACKER; et
- SLM 23 056/1 est un polydiméthylsiloxane à groupements aminoéthylaminopropyle et a,w-lauryle commercialisé par la société
   WACKER.

On constate que l'association du polyuréthanne amphiphile préféré de la présente invention (Acrysol 46) et d'une silicone non quaternisée ne permet pas la gélification du milieu aqueux mais donne lieu à une solution fluide ayant une viscosité peu différente de celle d'une solution du polyuréthanne seul. Par contre, l'addition de seulement 1 % en poids d'une des silicones à groupements ammonium quaternaire de la présente invention à une solution de polyuréthanne se traduit par une gélification satisfaisante de ce même milieu aqueux.

### Exemple 2

On a préparé un gel de soin ayant la composition suivante :

| | |
|---|---|
| Acrysol 46 | 2,5 % de matière active |
| Abil Quat 3272 | 1,5 % de matière active |
| parfum, colorant, conservateur | |
| Eau déminéralisée q. s. | 100 g |

### Exemple 3

On a préparé un gel de soin ayant la composition suivante :

| | |
|---|---|
| Acrysol 46 | 3 % de matière active |
| Sil Quat Q100 | 2 % de matière active |
| parfum, colorant, conservateur | |
| Eau déminéralisée q. s. | 100 g |

## Revendications

1. Composition cosmétique **caractérisée par le fait qu'**elle comprend, dans un support cosmétiquement acceptable,
(A) au moins un polyuréthanne associatif non ionique amphiphile et
(B) au moins une silicone à groupements ammonium quaternaire.

2. Composition cosmétique selon la revendication 1, **caractérisée en ce que** ledit polyuréthanne associatif non ionique (A) est un polyuréthanne de formule générale 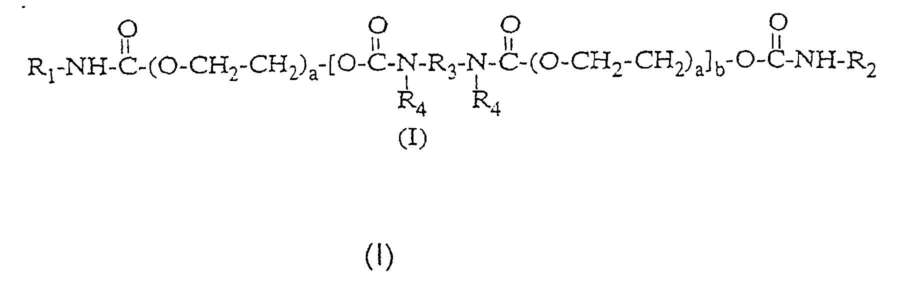 dans laquelle
au moins un des radicaux R₁ et R₂ représente un groupe alkyle supérieur en C₈₋₁₈ et l'autre, le cas échéant, un groupe alkyle inférieur en C₁₋₆, de préférence un groupe alkyle inférieur en C₁₋₄,
R₃ représente un radical hydrocarboné en C₄₋₃₆,
R₄ représente un atome d'hydrogène ou un radical alkyle en C₁₋₆,
a varie indépendamment de 90 à 600, et
b vaut de 1 à 4.

3. Composition cosmétique selon l'une quelconque des revendications 1 et 2, **caractérisée en ce que** le composant (A) est un polyuréthanne amphiphile dans lequel un seul des radicaux R₁ et R₂ représente une chaîne alkyle supérieure en C₈₋₁₈ et l'autre représente un groupe alkyle inférieur en C₁₋₆.

4. Composition cosmétique selon l'une quelconque des revendications 1 à 3, **caractérisée en ce que** le composant (A) est un polyuréthanne amphiphile dans lequel un des radicaux en a-W représente un groupe octadécyle et l'autre un groupe méthyle.

5. Composition cosmétique selon l'une quelconque des revendications 1 à 4, **caractérisée en ce que** le composant (A) est un polyuréthanne amphiphile obtenu par polycondensation d'hexaméthylèneisocyanate et de polyéthylèneglycol, et portant à ses extrémités respectivement en moyenne un résidu méthyle et un résidu octadécyle.

6. Composition cosmétique selon la revendication 1, **caractérisée par le fait que** le composant (A) se présente sous forme d'une solution ou suspension dans l'eau contenant de l'amidon soluble modifié par voie enzymatique.

7. Composition cosmétique selon l'une quelconque des revendications 1 à 5, **caractérisée en ce que** ladite silicone à groupements ammonium quaternaire est choisie parmi les silicones correspondant à une des formules générales suivantes
formules dans lesquelles 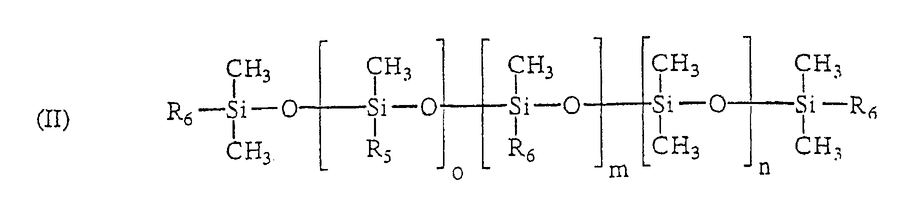 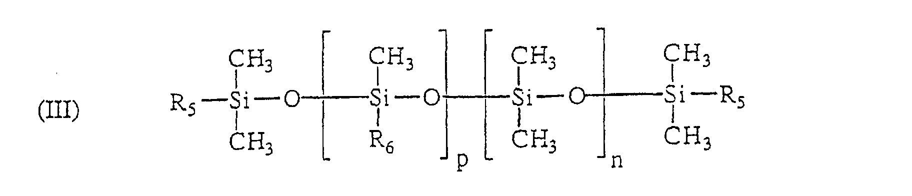 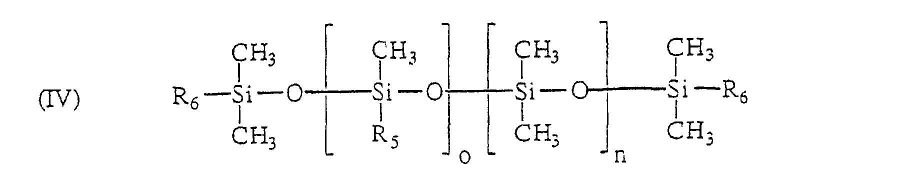 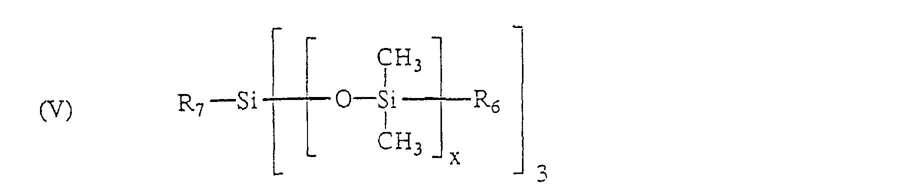
- les radicaux R₅, identiques ou différents, représentent des radicaux alkyle en C₁₋₃₀, linéaires ou ramifiés, ou phényle;
- les radicaux R₆, identiques ou différents, représentent un groupe -CₑH₂ₑ-O-(C₂H₄O)_{c}-(C₃H₆O)_{d}-R₈ ou -CₑH₂ₑ-O-(C₄H₈O)_{c}-R₈,
- les radicaux R₇, identiques ou différents, désignent un radical alkyle en C₁₋₁₂, linéaire ou ramifié, et de préférence le radical méthyle;
- les radicaux R₈, identiques ou différents, représentent un
radical 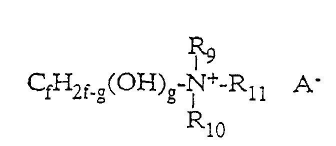
- les radicaux R₁₁ représentent indépendamment un radical alkyle en C₁₋₂₂ ou alcényle en C₂₋₂₂, linéaire ou ramifié, et portant éventuellement un ou plusieurs groupements OH, ou représentent un groupement CₕH₂ₕZᵢCOR₁₂;
- R₉, R₁₀ et R₁₂, identiques ou différents, représentent des radicaux alkyle en C₁₋₂₂ ou alcényle en C₂₋₂₂, linéaires ou ramifiés, portant éventuellement un ou plusieurs groupements OH, ou R₁₀ peut former avec une partie de R₁₁ un cycle imidazoline,
c varie de 0 à 50,
d varie de 0 à 50,
e et f varient indépendamment de 0 à 4,
m varie de 0 à 20,
n varie de 0 à 500,
o varie de 0 à 20,
p varie de 1 à 50,
x varie de 1 à 100,
g varie de 0 à 2,
h varie de 1 à 4
Z représente un atome d'oxygène ou d'azote,
i vaut 0 ou 1, et
A⁻ représente un ion halogénure ou un anion organique de type carboxylate, phosphate, alcanesulfate ou sulfate.

8. Composition cosmétique selon l'une quelconque des revendications 1 à 7, **caractérisée en ce que** les silicones à groupements ammonium quaternaire se présentent sous forme de solutions, de suspensions ou de dispersions dans l'eau.

9. Composition selon l'une quelconque des revendications 1 à 8, **caractérisée en ce qu'**elle contient le composant (A) à raison de 0,1 à 10 % en poids et, de préférence, de 0,5 à 5 % en poids en termes de matière active rapportée au poids total de la composition.

10. Composition selon l'une quelconque des revendications 1 à 9, **caractérisée en ce qu'**elle contient le composant (B) à raison de 0,01 à
10 % en poids, de préférence à raison de 0,1 à 5 % en poids de matière active par rapport au poids total de la composition.

11. Composition selon l'une quelconque des revendications 1 à 10, **caractérisé** en ce le rapport pondéral silicone/polyuréthanne amphiphile est compris entre 0,1 et 10.

12. Composition selon l'une quelconque des revendications 1 à 11 **caractérisée par le fait qu'**elle se présente sous forme d'un gel de coiffage ou d'un gel de soin capillaire non rincé.

13. Utilisation de l'association d'au moins un polyuréthanne associatif non ionique de formule (I) et d'au moins une silicone à groupements ammonium quaternaire en tant que système épaississant d'une composition cosmétique.

14. Procédé de traitement cosmétique des cheveux, **caractérisé** en ce l'on applique sur les cheveux la composition définie selon l'une quelconque des revendications 1 à 12 et en ce que l'on sèche les cheveux ainsi traités sans les rincer.

## Patentansprüche

1. Kosmetische Zusammensetzung, **dadurch gekennzeichnet, dass** sie in einem kosmetisch akzeptablen Träger
(A) mindestens ein amphiphiles nichtionisches assoziatives Polyurethan und
(B) mindestens ein Silicon mit quartären Ammoniumgruppen enthält.

2. Kosmetische Zusammensetzung nach Anspruch 1, **dadurch gekennzeichnet, dass** es sich bei dem nichtionischen assoziativen Polyurethan (A) um ein Polyurethan der folgenden allgemeinen Formel handelt: 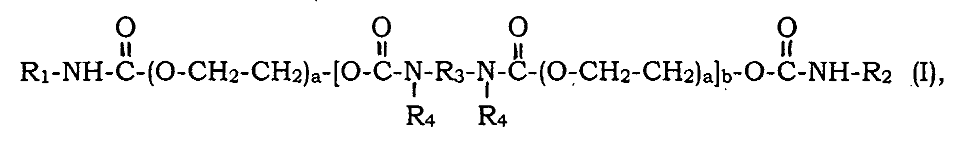 worin bedeuten:
- mindestens eine der Gruppen R₁ und R₂ eine höhere C₈₋₁₈-Alkylgruppe und die andere Gruppe gegebenenfalls eine niedere C₁₋₆-Alkylgruppe und vorzugsweise eine niedere C₁₋₄-Alkylgruppe,
- R₃ eine Kohlenwasserstoffgruppe mit 4 bis 36 Kohlenstoffatomen,
- R₄ Wasserstoff oder eine C₁₋₆-Alkylgruppe,
- die Indices a unabhängig voneinander Werte im Bereich von 90 bis 600 und
- b 1 bis 4.

3. Kosmetische Zusammensetzung nach einem der Ansprüche 1 und 2, **dadurch gekennzeichnet, dass** die Komponente (A) ein amphiphiles Polyurethan ist, worin nur eine der Gruppen R₁ und R₂ eine höhere C₈₋₁₈-Alkylgruppe bedeutet und die andere Gruppe eine niedere C₁₋₆-Alkylgruppe bedeutet.

4. Kosmetische Zusammensetzung nach einem der Ansprüche 1 bis 3, **dadurch gekennzeichnet, dass** die Komponente (A) ein amphiphiles Polyurethan ist, worin eine der Gruppen in α-ω-Stellung eine Octadecylgruppe und die andere Gruppe eine Methylgruppe bedeutet.

5. Kosmetische Zusammensetzung nach einem der Ansprüche 1 bis 4, **dadurch gekennzeichnet, dass** die Komponente (A) ein amphiphiles Polyurethan ist, das durch Polykondensation von Hexamethylenisocyanat und Polyethylenglykol erhalten wird und das an seinen Enden im Mittel eine Methylgruppe bzw. eine Octadecylgruppe aufweist.

6. Kosmetische Zusammensetzung nach Anspruch 1, **dadurch gekennzeichnet, dass** die Komponente (A) in Form einer Lösung oder Suspension in Wasser vorliegt, die auf enzymatischem Wege modifizierte lösliche Stärke enthält.

7. Kosmetische Zusammensetzung nach einem der Ansprüche 1 bis 5, **dadurch gekennzeichnet, dass** das Silicon mit quartären Ammoniumgruppen unter den Siliconen ausgewählt ist, die einer der folgenden allgemeinen Formeln entsprechen: 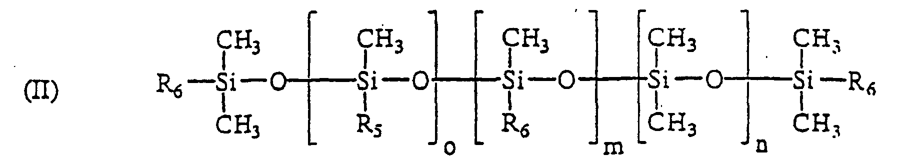 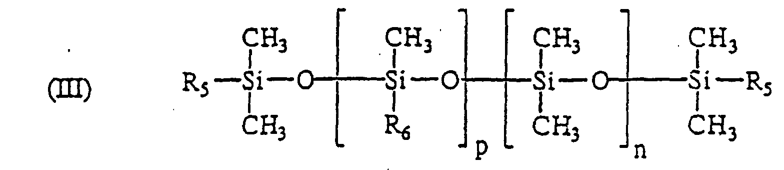 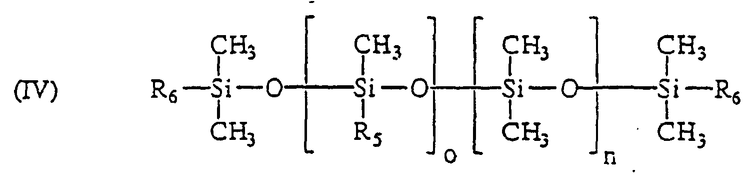 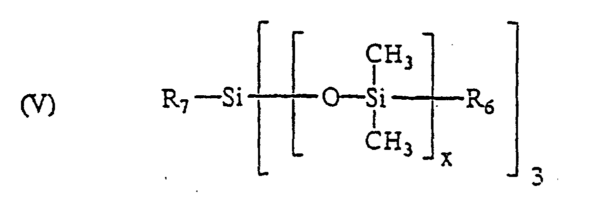 wobei in den Formeln:
- die Gruppen R₅, die identisch oder voneinander verschieden sind, geradkettige oder verzweigte C₁₋₃₀-Alkylgruppen oder Phenyl bedeuten,
- die Gruppen R₆, die identisch oder voneinander verschieden sind, eine Gruppe -CₑH₂ₑ-O-(C₂H₄O)_{c}-(C₃H₆O)_{d}-R₈ oder -CₑH₂ₑ-O-(C₄H₈O)_{c}-R₈ bedeuten,
- die Gruppen R₇, die identisch oder voneinander verschieden sind, eine geradkettige oder verzweigte C₁₋₁₂-Alkylgruppe und vorzugsweise Methyl bedeuten,
- die Gruppen R₈, die identisch oder voneinander verschieden sind, eine Gruppe 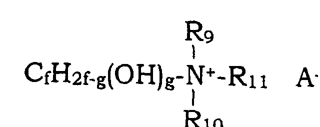 bedeuten,
- die Gruppen R₁₁ unabhängig voneinander eine geradkettige oder verzweigte C₁₋₂₂-Alkylgruppe oder C₂₋₂₂-Alkenylgruppe, die gegebenenfalls eine oder mehrere OH-Gruppen trägt, oder eine Gruppe CₕH₂ₕZᵢCOR₁₂ bedeuten,
- die Gruppen R₉, R₁₀ und R₁₂, die identisch oder voneinander verschieden sind, geradkettige oder verzweigte C₁₋₂₂-Alkylgruppen oder C₂₋₂₂-Alkenylgruppen bedeuten, die gegebenenfalls eine oder mehrere OH-Gruppen aufweisen, oder die Gruppe R₁₀ mit einem Teil von R₁₁ einen Imidazolinring bilden kann,
- c im Bereich von 0 bis 50 liegt,
- d im Bereich von 0 bis 50 liegt,
- e und f unabhängig voneinander Werte im Bereich von 0 bis 4 bedeuten,
- m im Bereich von 0 bis 20 liegt,
- n im Bereich von 0 bis 500 liegt,
- o im Bereich von 0 bis 20 liegt,
- p im Bereich von 1 bis 50 liegt,
- x im Bereich von 1 bis 100 liegt,
- g im Bereich von 0 bis 2 liegt,
- h im Bereich von 1 bis 4 liegt,
- Z ein Sauerstoffatom oder ein Stickstoffatom bedeutet,
- i 0 oder 1 bedeutet und
- A⁻ ein Halogenidion oder ein organisches Anion vom Typ Carboxylat, Phosphat, Alkansulfat oder Sulfat bedeutet.

8. Kosmetische Zusammensetzung nach einem der Ansprüche 1 bis 7, **dadurch gekennzeichnet, dass** die Silicone mit quartären Ammoniumgruppen in Form von Lösungen, Suspensionen oder Dispersionen in Wasser vorliegen.

9. Zusammensetzung nach einem der Ansprüche 1 bis 8, **dadurch gekennzeichnet, dass** sie die Komponente (A) in einem als Wirkstoff ausgedrückten Mengenanteil von 0,1 bis 10 Gew.-% und vorzugsweise von 0,5 bis 5 Gew.-%, bezogen auf das Gesamtgewicht der Zusammensetzung, enthält.

10. Zusammensetzung nach einem der Ansprüche 1 bis 9, **dadurch gekennzeichnet, dass** sie die Komponente (B) in einem als Wirkstoff ausgedrückten Mengenanteil von 0,01 bis 10 Gew.-% und vorzugsweise von 0,1 bis 5 Gew.-%, bezogen auf das Gesamtgewicht der Zusammensetzung, enthält.

11. Zusammensetzung nach einem der Ansprüche 1 bis 10, **dadurch gekennzeichnet, dass** das Gewichtsverhältnis Silicon/amphiphiles Polyurethan im Bereich von 0,1 bis 10 liegt.

12. Zusammensetzung nach einem der Ansprüche 1 bis 11, **dadurch gekennzeichnet, dass** sie in Form eines Frisiergels oder eines Haarpflegegels vorliegt, das nicht ausgespült wird.

13. Verwendung der Kombination mindestens eines nichtionischen assoziativen Polyurethans der Formel (I) und mindestens eines Silicons mit quartären Ammoniumgruppen als Verdickungssystem für eine kosmetische Zusammensetzung.

14. Verfahren zur kosmetischen Behandlung der Haare, **dadurch gekennzeichnet, dass** die in einem der Ansprüche 1 bis 12 definierte Zusammensetzung auf die Haare aufgebracht wird und dadurch, dass die so behandelten Haare ohne Spülen getrocknet werden.

## Claims

1. Cosmetic composition, **characterized in that** it comprises in a cosmetically acceptable medium,
(A) at least one amphiphilic nonionic associative polyurethane, and
(B) at least one silicone containing quaternary ammonium groups.

2. Cosmetic composition according to Claim 1, **characterized in that** the said nonionic associative polyurethane (A) is a polyurethane of general formula 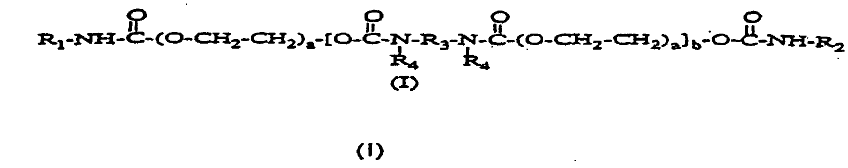 in which
at least one of the radicals R₁ and R₂ represents a C₈-C₁₈ higher alkyl group and the other, where appropriate, represents a C₁-C₆ lower alkyl group, preferably a C₁-C₄ lower alkyl group,
R₃ represents a C₄-C₃₆ hydrocarbon-based radical,
R₄ represents a hydrogen atom or a C₁-C₆ alkyl radical,
a ranges, independently, from 90 to 600, and
b is from 1 to 4.

3. Cosmetic composition according to either of Claims 1 and 2, **characterized in that** the component (A) is an amphiphilic polyurethane in which only one of the radicals R₁ and R₂ represents a C₈-C₁₈ higher alkyl chain and the other represents a C₁-C₆ lower alkyl group.

4. Cosmetic composition according to any one of Claims 1 to 3, **characterized in that** the component (A) is an amphiphilic polyurethane in which one of the α-ω radicals represents an octadecyl group and the other represents a methyl group.

5. Cosmetic composition according to any one of Claims 1 to 4, **characterized in that** the component (A) is an amphiphilic polyurethane obtained by polycondensation of hexamethylene isocyanate and polyethylene glycol, and bearing, on average, a methyl residue and an octadecyl residue at its ends, respectively.

6. Cosmetic composition according to Claim 1, **characterized in that** the component (A) is in the form of a solution or suspension in water containing enzymatically modified soluble starch.

7. Cosmetic composition according to any one of Claims 1 to 5, **characterized in that** the said silicone containing quaternary ammonium groups is chosen from silicones corresponding to one of the following general formulae 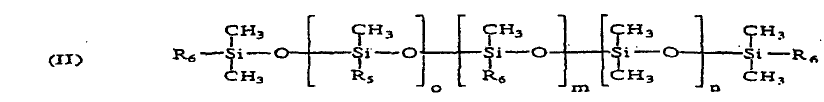 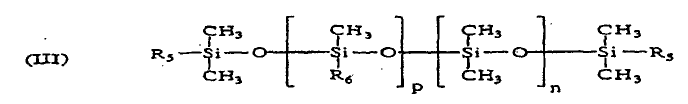 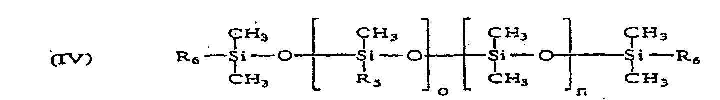 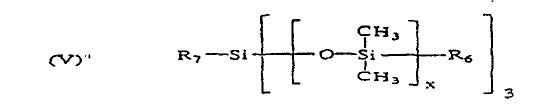 in which formulae:
- the radicals R₅, which may be identical or different, represent linear or branched C₁-C₃₀ alkyl radicals, or phenyl radicals;
- the radicals R₆, which may be identical or different, represent a group -CₑH₂ₑ-O-(C₂H₄O)_{c}-(C₃H₆O)_{d}-R₈ or -CₑH₂ₑ-O-(C₄H₈O)_{c}-R₈,
- the radicals R₇, which may be identical or different, denote a linear or branched C₁-C₁₂ alkyl radical and preferably a methyl radical;
- the radicals R₈, which may be identical or different, represent a radical 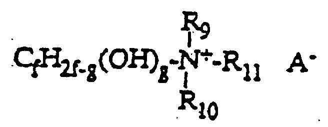
- the radicals R₁₁ represent, independently, a linear or branched C₁-C₂₂ alkyl or C₂-C₂₂ alkenyl radical, optionally bearing one or more OH groups; or represent a group CₕH₂ₕZᵢCOR₁₂;
- R₉, R₁₀ and R₁₂, which may be identical or different, represent linear or branched C₁-C₂₂ alkyl or C₂-C₂₂ alkenyl radicals, optionally bearing one or more OH groups, or R₁₀ can form, with a portion of R₁₁, an imidazoline ring,
c ranges from 0 to 50,
d ranges from 0 to 50,
e and f range, independently, from 0 to 4,
m ranges from 0 to 20,
n ranges from 0 to 500,
o ranges from 0 to 20,
p ranges from 1 to 50,
x ranges from 1 to 100,
g ranges from 0 to 2,
h ranges from 1 to 4,
Z represents an oxygen or nitrogen atom,
i is 0 or 1, and
A⁻ represents a halide ion or an organic anion of carboxylate, phosphate, alkanesulphate or sulphate type.

8. Cosmetic composition according to any one of Claims 1 to 7, **characterized in that** the silicones containing quaternary ammonium groups are in the form of solutions, suspensions or dispersions in water.

9. Composition according to any one of Claims 1 to 8, **characterized in that** it contains the component (A) in a proportion of from 0.1 to 10 % by weight, and preferably from 0.5 to 5 % by weight, in terms of active material relative to the total weight of the composition.

10. Composition according to any one of Claims 1 to 9, **characterized in that** it contains the component (B) in a proportion of from 0.01 to 10 % by weight, preferably in a proportion of from 0.1 to 5 % by weight, of active material relative to the total weight of the composition.

11. Composition according to any one of Claims 1 to 10, **characterized in that** the silicone/amphiphilic polyurethane weight ratio is between 0.1 and 10.

12. Composition according to any one of Claims 1 to 11, **characterized in that** it is in the form of a leave-in haircare gel or styling gel.

13. Use of the combination of at least one nonionic associative polyurethane of formula (I) and of at least one silicone containing quaternary ammonium groups as a system for thickening a cosmetic composition.

14. Cosmetic process for treating the hair, **characterized in that** the composition defined according to any one of Claims 1 to 12 is applied to the hair and **in that** the hair thus treated is dried without rinsing it.
